# EUROPÄISCHE PATENTSCHRIFT

(11) **EP 0 547 059 B1**
(45) Veröffentlichungstag und Bekanntmachung des Hinweises auf die Patenterteilung: **04.10.1995**
(21) Anmeldenummer: 91912932.0
(22) Anmeldetag: 28.07.1991
(51) Int. Cl.: C12P 21/08, A61K 39/385, C12N 9/66, G01N 33/573

(54) **PANKREAS-ELASTASE-1-SPEZIFISCHER ANTIKÖRPER, EIN VERFAHREN ZU SEINER GEWINNUNG UND EIN TESTKIT DER SOLCHE ANTIKÖRPER ENTHÄLT**
PANCREAS-ELASTASIS-1-SPECIFIC ANTIBODY, A PROCESS FOR OBTAINING IT AND A TEST KIT CONTAINING SUCH ANTIBODIES
ANTICORPS SPECIFIQUE A LA PANCREAS-ELASTASE-1, UN PROCEDE POUR SON OBTENTION ET UN KIT DE TEST CONTENANT DE TELS ANTICORPS

(30) Priorität: 28.07.1990 DE 4023972; 11.03.1991 DE 4107765
(43) Veröffentlichungstag der Anmeldung: 23.06.1993
(73) Patentinhaber: SCHEBO TECH MEDIZINISCH-BIOLOGISCHE FORSCHUNGSGESELLSCHAFT M.B.H, D-35435 Wettenberg (DE)
(72) Erfinder: SCHEEFERS, Hans, D-6301 Wettenberg 2 (DE); SCHEEFERS-BORCHEL, Ursula, D-6301 Wettenberg 2 (DE); SZIEGOLEIT, Andreas, D-6306 Langgöns (DE)
(74) Vertreter: Fritzsche, Thomas M., Dr.
(86) Internationale Anmeldenummer: DE9100606
(87) Internationale Veröffentlichungsnummer: WO9202630

(56) Entgegenhaltungen:
- ENZYME, Band 30, 30 Jänner 1983; A. MURATA, Seiten 29-37#
- CHEMICAL ABSTRACTS, Band 114, Nr. 11, 18 März 1991, Columbus, OH (US); S. HAYAKAWA, Nr. 97126d#
- CHEMICAL ABSTRACTS, Band 110, Nr. 25, 19 Juni 1989, Columbus, OH (US); K. KUNIO, Nr. 227738k#
- BIOL. CHEM. HOPPE-SEYLER, Band 368, Nr. 12, Dezember 1987; A. SZIEGOLEIT, Seiten 1613-1618#

## Beschreibung

Die Erfindung betrifft einen hochempfindlichen und selektiven Anti-Elastase 1-Antikörper, ein Verfahren zu seiner Herstellung sowie einen hochempfindlichen diagnostischen Testkit der solche Antikörper enthält.

Die entzündlichen Erkrankungen der Bauchspeicheldrüse nehmen in Industrieländern ständig zu (Rösch W., Deutsches Ärzteblatt 84: C-397 - 398; 1987). Die Erkrankung verläuft meist schubartig und kann schließlich zum völligen Verlust der Drüse führen. Dabei sind die akuten Schübe an starken Bauchschmerzen und Übelkeit erkennbar, die Zwischenphasen werden von den Patienten jedoch meist schmerzfrei erlebt. Sie gehen lediglich mit uncharakteristischen Verdauungsbeschwerden einher, sodaß sie schwer zu erkennen sind. Daher ist bei allen Verdauungsstörungen ein Verdacht auf eine chronische Bauchspeicheldrüsenerkrankung zu beachten.

Zur Diagnose der Pankreatitis im Labor wird bislang üblicherweise die Konzentration der Serumamylase bestimmt. Eine Erhöhung dieser Serumamylase ist jedoch auch bei anderen intraabdominellen Entzündungen, wie z. B. bei einer Darmperforation, bei Mumps oder auch bei Nierenversagen vorhanden. Darüberhinaus kann auch ein Anstieg der Serumamylasekonzentration nach der Gabe von Morphinen beobachtet werden. Eine weitere Möglichkeit in der Labordiagnostik besteht in der Bestimmung des Verhältnisses von Amylase zu Creatinin-Clearance, das bei der akuten Pankreatitis ansteigt. Leider normalisieren sich die bei der akuten Pankreatitis erhöhten Amylasewerte sehr rasch, sodaß bereits 48 h nach Erkrankungsbeginn bei einem Drittel der Patienten wieder Normalwerte gefunden werden (Eckfeldt, J. A. et al., Arch. Pathol. Lab. Med. 109, 316 - 319; 1985).

Eine weitere Diagnosemöglichkeit besteht in der Bestimmung der Lipase. Sowohl die Bestimmung der Lipase als auch der Amylase eignen sich jedoch nicht zur Erfassung der chronischen Pankreatitis. Diese läßt sich bislang nur unzureichend durch die Bestimmung der enzymatischen Aktivität des Pankreasenzyms Chymotrypsin im Stuhl nachweisen. Der Nachteil dieser Bestimmungsmethode liegt jedoch darin, daß lediglich ein geringer Teil des vom Pankreas ausgeschütteten Chymotrypsins im Stuhl nachweisbar ist, der darüberhinaus auch noch sehr starken Schwankungen unterworfen ist (Goldberg et al., Gut 10, 477 - 483; 1969). Dies macht die Festlegung von Normalwerten äußerst schwierig.

Aus A.Sziegoleit, Biochem. J. 219; 735-742; (1984) ist es bekannt, daß die Pankreaselastase 1 (E1), die auch Protease E genannt wird, ausschließlich im Pankreas gebildet wird und mit dem Verdauungssaft in das Duodenum abgesondert wird. Zur Überwindung der zuvor geschilderten Nachteile ist daher bereits versucht worden, die Elastase 1 im Stuhl zu bestimmen, da die Konzentration dieses Enzyms im Stuhl die exokrine Funktion der Bauchspeicheldrüse wesentlich besser darstellt als die Aktivität von Chymotrypsin (Sziegoleit A. et al., Clin. Biochem. 22, 85 - 89; 1989).

Es hat sich aber auch gezeigt, daß sich die akute Pankreatitis mittels der Bestimmung von E1 im Serum nachweisen läßt (Sziegoleit A. et al., Clin. Biochem. 22, 79 - 83; 1989).

Es ist bislang angenommen worden, daß im Gegensatz zu den übrigen Enzymen, E1 während der Darmpassage nicht oder nur unwesentlich abgebaut wird. Seine Konzentration im Stuhl zeigt somit den Funktionsgrad des exokrinen Pankreas an. Darüberhinaus wird in akuten Erkrankungsphasen der Drüse das Enzym auch in die Blutbahn überführt.

Zur Messung der Elastase 1 im Serum steht bereits ein radio-immunologischer Test zur Verfügung (Murata A. et al., Enzyme 30, 29 - 37; 1983, Elastase-1-RIA-Kit, Abbott Diagnostic).

Eine solche radiologische (RIA) Bestimmung hat jedoch den Nachteil, daß die radioaktiven Reagenzien nur eine begrenzte Haltbarkeit aufweisen und daher laufend nachsynthetisiert werden müssen. Darüberhinaus muß das radioaktive Material aufwendig entsorgt werden und die Messung von radioaktiven Stoffen benötigt besonders ausgebildetes Personal und eine spezielle Laborausrüstung. Außerdem ist es mit diesem Test nicht oder nur unzureichend möglich, die Konzentration von E1 im Stuhl zu bestimmen.

Die Erfindung hat daher zum Ziel, ein Testverfahren bereitzustellen, mit dem die menschliche Elastase 1 sowohl zur Diagnose der akuten als auch zur Diagnose der chronischen Pankreatitis durchgeführt werden kann und das sowohl für die Bestimmung der Elastase 1 im Serum als auch im Stuhl ausreichend empfindlich ist.

Dieses Ziel wird erfindungsgemäß mittels einem Antikörper erreicht, der gegen das Epitop mit der Aminosäuresequenz Thr-Met-Val-Ala-Gly-Gly-Asp-Ile-Arg gerichtet ist. Es hat sich nämlich überraschenderweise gezeigt, daß Antikörper, die gegen dieses Epitop der Humanelastase 1 gerichtet sind, besonders selektiv das Markerenzym erkennen und dabei andere Antigene diskriminieren.

Die Erfindung betrifft daher auch ein Verfahren zur Herstellung von Anti-Elastase-Antikörpern auf an sich bekannte Weise, das jedoch dadurch gekennzeichnet ist, daß man als Antigen das zuvor definierte Epitop verwendet. Erfindungsgemäß ist es auch möglich, Teile und Bruchstücke dieses Epitops zur Immunisierung bzw. zur Herstellung von Antikörpern zu verwenden, solange diese eine Immunantwort auslösen. Solche Fragmente sind sowohl auf synthetischem Wege als auch durch chemischen und/oder biologischen Abbau der Elastase 1 erhältlich. Im erfindingsgemäßen Verfahren hat es sich als zweckmäßig erwiesen, das Peptid oder das Teilpeptid, ggf. über einen Spacer, an einen Träger zu binden. Geeignete Träger sind dem Fachmann bekannt und sind insbesondere synthetische und natürliche Membranteile, Polyzucker oder Peptide bzw Proteine. Besonders bevorzugt sind wiederum Albumine und Hämocyanine. Die zu verwendenden Spacer sind dem Fachmann ebenfalls bekannt. Mit dem erfindungsgemäßen Epitop bzw.dessen Fragmenten ist es möglich sowohl selektive monoklonale als auch polyklonale Antikörper zu erhalten. Vorzugsweise werden paraffingängige Antikörper erhalten.

Antiseren, welche die erfindungsgemäßen Antikörper enthalten, werden dadurch gewonnen, daß man Versuchstiere mit hochgereinigter menschlicher Elastase 1 oder mit den Fragmenten dieses Enzyms immunisiert. Dabei werden Versuchstiere wie Mäuse, Ratten, Kaninchen, Ziegen oder Pferde in an sich bekannter Weise immunisiert und so Antiseren mit polyklonalen Antikörpern erhalten aus denen die erfindungsgemäßen Antikörper auf ebenfalls bekannte Weise erhältlich sind. Bevorzugte erfindungsgemäße Antikörper sind paraffingängig.

In einer bevorzugten Ausführungsform werden mittels dem erfindungsgemäßen Epitop in entsprechender Weise monoklonale Antikörper nach der Methode von G. Köhler und C. Milstein, (Nature 256, 495 - 497; 1975) gewonnen.

Ein weiterer Gegenstand der Erfindung ist daher ein monoklonaler Antikörper, der spezifisch mit E1 bindefähig ist. Ein solcher Antikörper ist erhältlich, indem man Mäuse oder Ratten mit hochgereinigter E1, bzw. dem erfindungsgemäß zu verwendenden Epitop, immunisiert, B-Lymphozyten aus der Milz der immunisierten Tiere mit Myelomzellen fusioniert, die gebildeten Hybridomazellen kloniert, die Hybridomazellen, die E1 bindungsfähige Antikörper sezernieren, isoliert, kloniert und züchtet und den durch sie gebildeten monoklonalen Antikörper gewinnt.

Besonders bevorzugt wird ein Zellinie verwendet, die selbst kein Immunoglobulin produziert.

Die erfindungsgemäß erhältlichen monoklonalen Antikörper reagieren nicht mit anderen Substanzen, sondern sind für El spezifisch. Die erfindungsgemäßen monoklonalen Antikörper sind vorzugsweise paraffingängig.

Bevorzugte erfindungsgemäße Antikörper sind aus den Hybridoma-Zellinien erhältlich, die bei der European Collection of Animal Cell Cultures (ECACC), Vaccine Research and Production Laboratory, Public Health and Laboratory Service, Center for Applied Microbiology and Research, Porton Down, GB-Salisbury, Wiltshire SP4 OJG, am 21. Dezember 1990 hinterlegt worden sind, und die die Anmeldenummern 90 121 90 6 und 90 121 90 7 erhalten haben. Beide aus diesen Zellinien erhältliche Antikörper sind paraffingängig.

Ein weiterer Gegenstand der Erfindung ist die Verwendung des erfindungsgemäßen E1-spezifischen Antikörpers zur qualitativen und/oder quantitativen Bestimmung von E1. Es ist somit möglich, unter Verwendung des Antikörpers die Elastase 1 spezifisch in Körperflüssigkeiten und im Stuhl nachzuweisen. Die Erfindung betrifft daher auch einen Testkit der die erfindungsgemäßen Antikörper enthält, und zwar insbesondere immunologische Testkits zur Diagnose und zur Verlaufskontrolle der chronischen Pankreatitis, der akuten Pankreatitis und der Mucoviscidose in Körperflüssigkeiten und/oder im Stuhl. Zweckmäßige Körperflüssigkeiten sind dabei Blut, Plasma und Serum.

Bei Versuchen zum Nachweis von E1 im Blut, Plasma, Serum oder Stuhl wurden indirekte, kompetitive- und Sandwich-ELISA eingesetzt. Es hat sich jedoch gezeigt, daß ein Sandwich-ELISA für die schnelle Diagnostik bei großem Probenumfang am geeignetesten ist, da dieser von anderen nicht kalkulierbaren Serumfaktoren unabhängig ist. Hierfür sind mindestens zwei verschiedene monoklonale Antikörper nötig, die gegen unterschiedliche Epitope des Enzyms gerichtet sind.

Mit solchen Tests lassen sich Enzymverschiebungen z. B. im Serum oder Stuhl, insbesondere das Auftreten dieser Verschiebungen bei Veränderungen des Pankreas-Status, nachweisen.

Bestimmungsverfahren nach dem Prinzip des Immunoassays sind weit verbreitet. Vorteil dieser Bestimmungsmethode ist ihre Genauigkeit und Schnelligkeit (große Sicherheit und Probendurchsatz) sowie die Möglichkeit, sehr geringe Substanzmengen nachweisen zu können (im Nanogramm-Bereich). Zur Durchführung der Bestimmung sind verschiedene Verfahrensvarianten möglich, sowohl mit homogenen als auch mit heterogenen Phasen. Bei der Ausführungsform mit heterogener Phase wird einer der Rezeptoren an einen Träger gebunden. Beim Sandwich-Verfahren wird beispielsweise ein erster Antikörper als Rezeptor, bzw. als sog. Fänger oder Catcher an den Träger gebunden, die Testlösung zugegeben, wobei das in der Testlösung zu bestimmende Antigen von den Rezeptoren aus der Testlösung herausgefischt und gebunden wird. Anschließend wird ein zweiter, markierter Antikörper zugegeben, der spezifisch mit dem Antigen oder Antigen-Rezeptorkomplex reagiert. Mittels der Markierung des zweiten Antikörpers kann dann mit Hilfe einer Eichlösung (isolierte, gereinigte Human-Elastase 1) die Menge an Antigen bestimmt werden.

In einer weiteren bevorzugten erfindungsgemäßen Ausführungsform wird ein erster Antikörper als Rezeptor an einer Trägermatrix mit einer Membran-, Gewebs- oder Vliesstruktur derart gebunden, daß er nicht wie üblicherweise den Boden der Vertiefung einer Elisa-Immunoplatte darstellt, sondern in der Matrix gebunden vorliegt. Bevorzugte Matrizes sind microporöse Flachmembranen oder Hohlfaser-Mebranen, die in einer besonderen Ausführungsform mit Ionenaustauschergruppen versehen sind. Hierzu werden vorzugsweise solche microporösen Flachmembranen verwendet, wie sie z.B. von der Pall Corp., New Jersey, USA vertrieben werden. Die erfindungsgemäß zu verwendenden Hohlfaser-Membranen sind ebenfalls im Handel erhältlich und werden z.B. von Sepracor Inc., Ma., USA vertrieben. Mittels solcher Trägermaterialien ist es möglich besonders schnelle und unkomplizierte Nachweisverfahren bereitzustellen.

Für dieses allgemeine Prinzip gibt es viele Variationsmöglichkeiten. So kann beispielsweise eine Bestimmung mit drei Rezeptoren erfolgen, wobei einer der drei Rezeptoren in heterogener Phase vorliegt und die anderen beiden Rezeptoren löslich sind. Einer der beiden löslichen Rezeptoren ist markiert, während der andere unmarkiert ist. Der lösliche Rezeptor ist dann gegen den nicht markierten Rezeptor gerichtet.

Die Verwendung der erfindungsgemäßen E1 spezifischen Antikörper zur selektiven quantitativen Bestimmung von E1 nach dem Prinzip des Immunoassays erfolgt durch Inkubation mit mindestens zwei verschiedenen Rezeptoren. Beide Rezeptoren, z. B. monoklonale Antikörper, müssen spezifisch für E1 sein, welches über jeweils unterschiedliche Epitope (Bindungsstellen) gebunden werden muß.

Einer der beiden Rezeptoren wird an eine Festphase gebunden. Die Bindung an die Festphase erfolgt in üblicher Weise und ist dem Fachmann bekannt. Weiterhin wird mindestens ein weiterer Rezeptor verwendet, der in löslicher Form vorliegt.

Dieser weitere Rezeptor trägt eine Markierung. Werden mehrere weitere Rezeptoren R2 verwendet, so trägt nur einer von diesen eine Markierung. Die Markierung des Rezeptors erfolgt in an sich üblicher Weise und ist dem Fachmann bekannt.

Im erfindungsgemäßen Testkit erfolgt die Markierung in an sich bekannter Weise, insbesonders durch eine radioaktive Markierung, durch-eine Bindung von Biotin (Biotin/Avidin), durch ein eine meßbare Reaktion auslösendes Enzym, bzw. durch eine chemilumineszente oder fluoreszierende Verbindung. Besonders bevorzugt ist die Markierung mit einem Enzym, insbesondere mit Peroxidase oder Phosphatase. Die Markierung mit einem Enzym erlaubt in einer besonderen Ausführungsform auch, diesen Antikörper in einem zweiten Enzymamplifikationssystem einzusetzen (Stanley, C. J., Paris, F., Plumb, A., Webb, A. Johannsson, A. American Biotechnology Laboratory: May/June; 1985; Self, C. H., J. Immunol. Meth.; 1985).

In einer besonders bevorzugten Ausführungsform des Verfahrens wird nun entweder ein unspezifisch mit E1 bindefähiger Rezeptor oder bevorzugt ein spezifisch mit E1 bindefähiger Rezeptor an eine Festphase gebunden. Dieser an die Festphase gebundene Rezeptor wird anschließend mit der Lösung, die das zu bestimmende E1 enthalten soll und einem Antikörper inkubiert, der spezifisch mit E1 bindefähig ist, in einer löslichen Form vorliegt und eine Markierung trägt.

Ist der an die Festphase gebundene Rezeptor ein unspezifisch mit E1 bindefähiger, so lagern sich an der Festphase nicht nur E1, sondern auch andere Antigene an. Der zweite Antikörper, der spezifisch mit E1 bindefähig ist, lagert sich jedoch nur an E1, sodaß nur E1-Moleküle spezifisch einen markierten Antikörper tragen, während die anderen Antigene nicht markiert werden. Auf diese Weise ist es nach Trennung der festen von der flüssigen Phase möglich, über die Messung der Markierung den Gehalt an E1 zu bestimmen.

Wird an die Festphase ein an E1 spezifisch bindefähiger erster Rezeptor fixiert, so wird an der Festphase spezifisch nur E1 gebunden. Bei der Inkubation mit dem löslichen, E1-spezifischen zweiten Rezeptor bzw. Antikörper reagiert auch dieser ausschließlich mit E1. Da somit praktisch keine Bindung anderer Antigene an die Festphase erfolgt, ist dieses Verfahren hoch spezifisch und erlaubt daher sehr genaue Bestimmungen. Dabei wird an der Festphase selektiv E1 gebunden, andere Antigene bleiben in Lösung. Weiterhin lagert sich an E1 der lösliche, markierte, mit E1 bindefähige Antikörper an. Nach Trennung der festen von der flüssigen Phase kann wiederum über die Markierung der Gehalt an E1 sehr genau bestimmt werden. In einer besonders bevorzugten Ausführungsform wird zur weiteren Erhöhung der Selektivität ein dritter Antikörper zugegeben, der gegen den zweiten Antikörper gerichtet ist und der die Markierung trägt.

Weitere, dem Fachmann bekannte Verfahrensvarianten mit drei Rezeptoren sind ebenfalls unter Verwendung der spezifisch mit E1 bindefähigen Antikörper möglich und bedürfen hier keiner weiteren Ausführungen.

Von den für die Durchführung des erfindungsgemäßen Verfahrens verwendeten Antikörpern ist vorzugsweise zumindest einer ein monoklonaler Antikörper. In einer bevorzugten Ausführungsform werden nur monoklonale Antikörper als Rezeptoren verwendet.

Der E1-spezifisch-bindefähige Antikörper kann entweder an die Festphase gebunden vorliegen oder aber als löslicher, markierter oder unmarkierter Rezeptor verwendet werden. Vorzugsweise ist dieser Rezeptor ein monoklonaler Antikörper. Besonders bevorzugt sind alle verwendeten Rezeptoren monoklonale Antikörper

Das erfindungsgemäße Verfahren, sowie der Testkit sind besonders für automatisierte Analysensyteme geeignet, insbesonders für solche Systeme, die auf einem Biosensor basieren und von der Chiptechnologie Gebrauch machen.

Die Erfindung wird durch die folgenden Beispiele näher erläutert:

### Beispiel 1

### a) Herstellung monoklonaler Antikörper mittels hochgereinigter E1:

Hochgereinigte menschliche E1, deren Gewinnung aus menschlichem Pankreas beschrieben ist (Sziegoleit A., Purification and Characterization of a cholesterol binding protein from human pancreas. Biochem. J. 207:573-582;1982), wird in PBS gelöst und zu gleichen Teilen mit Freund's-Adjuvans gemischt. Jeweils 100 µg dieses Gemisches werden in 6 bis 8 Wochen alte Balb/c Mäuse i.p. und s.c. injiziert. Diese Injektionen werden zweimal im Abstand von 3 bis 4 Wochen wiederholt. Nach obengenanntem Schema mit hochgereinigter E1 immunisierte Mäuse erhalten 3 Tage vor der Entnahme der Milz eine i.v. Injektion von 100 µg hochgereinigter E1, welche in PBS gelöst ist.

Etwa 100 Mill. Zellen von der Milz einer immunisierten Maus werden mit 50 Mill. Myelomazellen (x 63-Sp8-653, eine Zellinie, die kein Immunglobulin synthetisiert; zu beziehen bei The Salk Institute, Cell Distribution Center, San Diego CA 92112, USA) in Gegenwart von Polyethylenglykol (MG 3400) fusioniert. Fusionierte Zellen werden auf 8 Platten, die jeweils 24 Vertiefungen enthalten, ausgesät. Jede dieser Vertiefungen enthält 50 Mill. Milzzellen unimmunisierter syngener Mäuse in Nährmedium, welches Hypoxantin, Aminopterin und Thymidin enthält.

Die Antikörper enthaltenden Überstände dieser fusionierten Zellen (Hybridoma) werden 10 bis 14 Tage später mittels ELISA, Westernblott, Gefrierschnitt, Paraffinschnitt bezüglich ihrer Spezifität gegen hochgereinigte, menschliche E1 getestet.

Um monoklonale Antikörper zu erhalten, die nur gegen E1 gerichtet sind, werden Hybridomazellen, deren Überstände keine gegen andere Antigene gerichteten Antikörper enthalten, zweimal kloniert.

### b) Herstellung monoklonaler Antikörper mittels einem immunogenen Peptid:

Es wird mittels einer Festphasensynthese nach Merrifield das Peptid mit der Aminosäuresequenz Thr-Met-Val-Ala-Gly-Gly-Asp-Ile-Arg hergestellt und wie zuvor beschrieben in 6 bis 8 Wochen alte Balb/c Mäuse injiziert. Durch die zuvor beschriebene Vorgehensweise werden monoklonale Antikörper erhalten, die gegen menschliche Elastase 1 sowohl aus Stuhl als auch aus Serum hochspezifisch sind.

### Bestimmung von E1 in Plasma mit mononoklonalen E1-spezifischen Antikörpern.

### Beispiel 2

Es wird E1 im Plasma mit einem ELISA bestimmt. Hierzu werden gemäß Beispiel 1 erhaltene monoklonale, E1-spezifische Antikörper, in PBS, pH 7,2 gelöst und, an Polystyrol als Träger immobilisiert. Nach einem Waschschritt wird verdünntes Plasma oder Serum, welches E1 enthält, dazugegeben. Das Plasma oder Serum wird in einem Puffer der PBS, 5mmol EDTA und 0,2% Tween 20 enthält, verdünnt.

Nach einem Waschschritt in PBS, 0,2% Tween 20, wird die an den Antikörper gebundene E1 mit einem polyklonalen Antikörper, der auch E1 bindet und an den Phosphatase gekoppelt ist, der gelöst ist in PBS, das 0,2% Tween 20 enthält und einen pH von 7,2 hat, eine Stunde bei Raumtemperatur inkubiert. Nach einem erneuten Waschschritt wird durch Zugabe von p-Nitrophenylphosphatdinatriumhexahydrat eine Änderung der optischen Dichte in den Reaktionsgefäßen gemessen, in denen die monoklonalen Antikörper mit E1 reagiert haben.

### Bestimmung von E1 in Plasma mit zwei verschiedenen E1-spezifischen Antikörpern

### Beispiel 3

Ein monoklonaler Antikörper, der gegen E1 gerichtet ist, wird, wie in Beispiel 2 beschrieben, an einen Träger fixiert. Nach einem Waschschritt wird Serum bzw. Plasma, das E1 enthält, mit dem monokonalen Antikörper unter denselben Bedingungen wie im Beispiel 2 inkubiert. Nach einem weiteren Waschschritt wird die Bindung von E1 durch einen zweiten erfindungsgemäßen E1-spezifischen monoklonalen Antikörper detektiert. Dieser zweite E1-spezifische Antikörper trägt Peroxydase kovalent gebunden. Nach einem Waschschritt wird nach Zugabe von ABTS als Substrat für Peroxydase eine Änderung der optischen Dichte gemessen.

### Beispiel 4

Es wird wie in Beispiel 3 beschrieben verfahren, jedoch wird an den zweiten E1-spezifischen Antikörper statt eines Enzyms Biotin gekoppelt. Vor Substratzugabe wird dann Peroxidase-konjugiertes Avidin oder Peroxydase-konjugiertes Streptavidin zugesetzt. Die so gewählte Bestimmung von E1 erlaubt ganz spezifisch nur E1 zu bestimmen. Andere Antigene, die in der Lösung enthalten sind, stören die erfindungsgemäße Bestimmung der E1 nicht.

## Patentansprüche (Patentansprüche für folgende(n) Vertragsstaat(en): AT, BE, CH, DE, DK, FR, GB, IT, LI, LU, NL, SE)

1. Verfahren zur Gewinnung von mono und/oder polyklonalen Anti-Elastase-1-Antikörpern, die mit Elastase 1 in Körperflüssigkeiten und im Stuhl reagieren, durch Immunisierung auf an sich bekannte Weise **dadurch gekennzeichnet,** daß man ein Antigen verwendet, das die Aminosäuresequenz Thr-Met-Val-Ala-Gly-Gly-Asp-Ile-Arg oder immunologisch wirksame Teilpeptide davon aufweist.

2. Verfahren nach Anspruch 1, **dadurch gekennzeichnet,** daß man ein synthetisches Teilpeptid verwendet.

3. Verfahren nach einem der Ansprüche 1 oder 2, **dadurch gekennzeichnet,** daß man ein Teilpeptid verwendet, das über einen Spacer an einen Träger gebunden ist.

4. Verfahren nach Anspruch 3, **dadurch gekennzeichnet,** daß man als Träger ein Peptid verwendet.

5. Verfahren nach einem der Ansprüche 3 oder 4, **dadurch gekennzeichnet,** daß man als Träger ein Albumin oder ein Hämocyanin verwendet.

6. Hybridoma-Zellinie mit der Hinterlegungsnummer ECACC 90 121 90 6.

7. Hybridoma-Zellinie mit der Hinterlegungsnummer ECACC 90 121 90 7.

8. Anti-Elastase 1-Antikörper, erhältlich nach einem der Verfahren der Ansprüche 1 bis 5.

9. Anti-Elastase 1-Antikörper, erhältlich aus den Hybridoma-Zellinien mit den Hinterlegungsnummern ECACC 90 121 90 6 und ECACC 90 121 90 7.

10. Immunologischer Testkit, enthaltend zumindest einen Antikörper nach einem der Ansprüche 8 oder 9.

11. Immunologischer Testkit nach Anspruch 10 zur Diagnose und Verlaufskontrolle der chronischen Pankreatitis, der akuten Pankreatitis und der Mucoviscidose in Körperflüssigkeiten und/oder im Stuhl.

## Patentansprüche (Patentansprüche für folgende(n) Vertragsstaat(en): ES, GR)

1. Verfahren zur Gewinnung von mono-und/oder polyklonalen Anti-Elastase-1-Antikörpern, die mit Elastase 1 in Körperflüssigkeiten und im Stuhl reagieren, durch Immunisierung auf an sich bekannte Weise **dadurch gekennzeichnet,** daß man ein Antigen verwendet, das die Aminosäuresequenz Thr-Met-Val-Ala-Gly-Gly-Asp-Ile-Arg oder immunologisch wirksame Teilpeptide davon aufweist.

2. Verfahren nach Anspruch 1, **dadurch gekennzeichnet,** daß man ein synthetisches Teilpeptid verwendet.

3. Verfahren nach einem der Ansprüche 1 oder 2, **dadurch gekennzeichnet,** daß man ein Teilpeptid verwendet, das über einen Spacer an einen Träger gebunden ist.

4. Verfahren nach Anspruch 3, **dadurch gekennzeichnet,** daß man als Träger ein Peptid verwendet.

5. Verfahren nach einem der Ansprüche 3 oder 4, **dadurch gekennzeichnet,** daß man als Träger ein Albumin oder ein Hämocyanin verwendet.

6. Hybridoma-Zellinie mit der Hinterlegungsnummer ECACC 90 121 90 6.

7. Hybridoma-Zellinie mit der Hinterlegungsnummer ECACC 90 121 90 7.

8. Anti-Elastase 1-Antikörper, erhältlich nach einem der Verfahren der Ansprüche 1 bis 5.

9. Anti-Elastase 1-Antikörper, erhältlich aus den Hvbridoma-Zellinien mit den Hinterlegungsnummern ECACC 90 121 90 6 und ECACC 90 121 90 7.

10. Immunologischer Testkit, enthaltend zumindest einen Antikörper nach einem der Ansprüche 8 oder 9.

11. Immunologischer Testkit nach Anspruch 10 zur Diagnose und Verlaufskontrolle der chronischen Pankreatitis, der akuten Pankreatitis und der Mucoviscidose in Körperflüssigkeiten und/oder im Stuhl.

12. Verfahren zur Herstellung eines immunologischen Testkits, **dadurch gekennzeichnet,** daß man Antikörper verwendet, die nach einem der in den Ansprüchen 1 bis 5 definierten Verfahren und/oder aus den Hybridoma-Zellinien mit den Hinterlegungsnummern ECACC 90 121 90 6 und ECACC 90 121 90 7 erhältlich sind.

## Claims (Claims for the following Contracting State(s): AT, BE, CH, LI, DE, DK, FR, GB, IT, LU, NL, SE)

1. Process for obtaining mono- and/or polyclonal anti-elastase 1 antibodies, which react with elastase 1 in bodily fluids and in stools, by immunizing in a manner known per se, characterised in that an antigen is used having the amino acid sequence Thr-Met-Val-Ala-Gly-Gly-Asp-Ile-Arg or immunologically active partial peptides thereof.

2. Process according to claim 1, characterised in that a synthetic partial peptide is used.

3. Process according to one of claims 1 or 2, characterised in that a partial peptide is used which is bound to a carrier by means of a spacer.

4. Process according to claim 3, characterised in that a peptide is used as the carrier.

5. Process according to one of claims 3 or 4, characterised in that an albumin or a haemocyanin is used as the carrier.

6. Hybridoma cell line having the file number ECACC 90 121 90 6.

7. Hybridoma cell line having the file number ECACC 90 121 90 7.

8. Anti-elastase 1 antidbody obtainable according to one of the processes of claims 1 to 5.

9. Anti-elastase 1 antibody obtainable from the hybridoma cell lines having the file numbers ECACC 90 121 90 6 and ECACC 90 121 90 7.

10. Immunological test kit containing at least one antibody according to one of claims 8 or 9.

11. Immunological test kit according to claim 10 for the diagnosis and course monitoring of chronic pancreatitis, acute pancreatitis and mucoviscidosis in bodily fluids and/or stools.

## Claims (Claims for the following Contracting State(s): ES, GR)

1. Process for obtaining mono- and/or polyclonal anti-elastase 1 antibodies, which react with elastase 1 in bodily fluids and in stools, by immunizing in a manner known per se, characterised in that an antigen is used having the amino acid sequence Thr-Met-Val-Ala-Gly-Gly-Asp-Ile-Arg or immunologically active partial peptides thereof.

2. Process according to claim 1, characterised in that a synthetic partial peptide is used.

3. Process according to one of claims 1 or 2, characterised in that a partial peptide is used which is bound to a carrier by means of a spacer.

4. Process according to claim 3, characterised in that a peptide is used as the carrier.

5. Process according to one of claims 3 or 4, characterised in that an albumin or a haemocyanin is used as the carrier.

6. Hybridoma cell line having the file number ECACC 90 121 90 6.

7. Hybridoma cell line having the file number ECACC 90 121 90 7.

8. Anti-elastase 1 antidbody obtainable according to one of the processes of claims 1 to 5.

9. Anti-elastase 1 antibody obtainable from the hybridoma cell lines having the file numbers ECACC 90 121 90 6 and ECACC 90 121 90 7.

10. Immunological test kit containing at least one antibody according to one of claims 8 or 9.

11. Immunological test kit according to claim 10 for the diagnosis and course monitoring of chronic pancreatitis, acute pancreatitis and mucoviscidosis in bodily fluids and/or stools.

12. Process for preparing an immunological test kit, characterized by the use of antibodies obtainable by the processes defined in claims 1 to 5 and/or by the hybridoma cell lines having the file numbers ECACC 90 121 90 6 and ECACC 90 121 90 7.

## Revendications (Revendications pour l'(les) Etat(s) contractant(s) suivant(s): AT, BE, CH, DE, FR, GB, IT, LI, LU, NL, SE)

1. Procédé d'obtention d'anticorps anti-élastase 1 mono- et/ou polyclonaux, qui réagissent avec l'élastase 1 dans les liquides corporels et dans les selles, par immunisation de façon connue en soi, caractérisé en ce que l'on utilise un antigène contenant la séquence d'aminoacides Thr-Met-Val-Ala-Gly-Gly-Asp-Ile-Arg ou des fragments peptidiques à activité immunologique de cette séquence.

2. Procédé selon la revendication 1, caractérisé en ce que l'on utilise un fragment peptidique synthétique.

3. Procédé selon l'une des revendications 1 ou 2, caractérisé en ce que l'on utilise un fragment peptidique lié à un support par l'intermédiaire d'un espaceur.

4. Procédé selon la revendication 3, caractérisé en ce que l'on utilise un peptide comme support.

5. Procédé selon l'une des revendications 3 ou 4, caractérisé en ce que l'on utilise une albumine ou une hémocyanine comme support.

6. Lignée cellulaire d'hybridome ayant le numéro de dépôt ECACC 90 121 90 6.

7. Lignée cellulaire d'hybridome ayant le numéro de dépôt ECACC 90 121 90 7.

8. Anticorps anti-élastase 1, pouvant être obtenus par l'un des procédés des revendications 1 à 5.

9. Anticorps anti-élastase 1, pouvant être obtenus à partir des lignées cellulaires d'hybridomes ayant les numéros de dépôt ECACC 90 121 90 6 et ECACC 90 121 90 7.

10. Trousse d'analyse immunologique, contenant au moins un anticorps selon l'une des revendications 8 ou 9.

11. Trousse d'analyse immunologique selon la revendication 10 pour le diagnostic et le suivi de l'évolution de la pancréatite chronique, de la pancréatite aiguë et de la mucoviscidose dans les liquides corporels et/ou dans les selles.

## Revendications (Revendications pour l'(les) Etat(s) contractant(s) suivant(s): GR, ES)

1. Procédé d'obtention d'anticorps anti-élastase 1 mono- et/ou polyclonaux, qui réagissent avec l'élastase 1 dans les liquides corporels et dans les selles, par immunisation de façon connue en soi, caractérisé en ce que l'on utilise un antigène contenant la séquence d'aminoacides Thr-Met-Val-Ala-Gly-Gly-Asp-Ile-Arg ou des fragments peptidiques à activité immunologique de cette séquence.

2. Procédé selon la revendication 1, caractérisé en ce que l'on utilise un fragment peptidique synthétique.

3. Procédé selon l'une des revendications 1 ou 2, caractérisé en ce que l'on utilise un fragment peptidique lié à un support par l'intermédiaire d'un espaceur.

4. Procédé selon la revendication 3, caractérisé en ce que l'on utilise un peptide comme support.

5. Procédé selon l'une des revendications 3 ou 4, caractérisé en ce que l'on utilise une albumine ou une hémocyanine comme support.

6. Lignée cellulaire d'hybridome ayant le numéro de dépôt ECACC 90 121 90 6.

7. Lignée cellulaire d'hybridome ayant le numéro de dépôt ECACC 90 121 90 7.

8. Anticorps anti-élastase 1, pouvant être obtenus par l'un des procédés des revendications 1 à 5.

9. Anticorps anti-élastase 1, pouvant être obtenus à partir des lignées cellulaires d'hybridomes ayant les numéros de dépôt ECACC 90 121 90 6 et ECACC 90 121 90 7.

10. Trousse d'analyse immunologique, contenant au moins un anticorps selon l'une des revendications 8 ou 9.

11. Trousse d'analyse immunologique selon la revendication 10 pour le diagnostic et le suivi de l'évolution de la pancréatite chronique, de la pancréatite aiguë et de la mucoviscidose dans les liquides corporels et/ou dans les selles.

12. Procédé de préparation d'une trousse d'analyse immunologique, caractérisé en ce que l'on utilise des anticorps qui peuvent être obtenus par l'un des procédés définis dans les revendications 1 à 5 et/ou à partir des lignées cellulaires d'hybridomes ayant les numéros de dépôt ECACC 90 121 90 6 et ECACC 90 121 90 7.
